(19) Europäisches Patentamt
European Patent Office
Office européen des brevets

(11) Publication number: **0 006 208**
**B1**

(12) # EUROPEAN PATENT SPECIFICATION

(45) Date of publication of patent specification: **02.12.81**

(51) Int. Cl.³: **C 07 D 209/88,**
A 61 K 31/40

(21) Application number: **79101880.7**

(22) Date of filing: **11.06.79**

(54) **Process for the preparation of carbazoles and an intermediate therefor.**

(30) Priority: **12.06.78 US 914465**

(43) Date of publication of application:
**09.01.80 Bulletin 80/1**

(45) Publication of the grant of the European patent:
**02.12.81 Bulletin 81/48**

(84) Designated Contracting States:
**AT CH LU NL SE**

(56) References cited:
**DE - A - 2 141 640**
**DE - A - 2 337 340**
**US - A - 3 671 544**
**US - A - 3 868 387**

(73) Proprietor: **F. HOFFMANN-LA ROCHE & CO.**
**Aktiengesellschaft**
**CH-4002 Basel (CH)**

(72) Inventor: **Berger, Leo**
**7 The Parkway**
**Montclair New Jersey (US)**
Inventor: **Scott, John William**
**81 Edgemont Road**
**Upper Montclair New Jersey (US)**

(74) Representative: **Lederer, Franz, Dr. et al,**
**Patentanwälte Dr. Franz Lederer Reiner F. Meyer**
**Lucile-Grahn-Strasse 22**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 006 208 B1

Courier Press, Leamington Spa, England.

# Process for the preparation of carbazoles and an intermediate therefor

The invention relates to a process for the preparation of carbazole derivatives of the formula

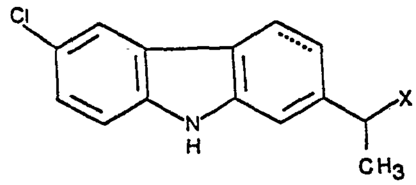

I

wherein X is hydroxymethyl or a grouping $COOR^1$, $R^1$ is hydrogen or lower alkyl, and the dotted line denotes an optional C—C bond, and, where X is carboxy, salts thereof with bases, comprising

a) reacting 6 - chloro - 1,2,3,4 - tetrahydro - carbazole - 2 - one with a trialkyl - $\alpha$ - methylphosphonoacetate of the formula

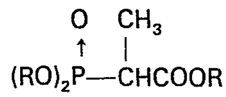

II

wherein R is lower alkyl, in the presence of a strong base,

b) if desired, saponifying the resulting ester of the formula

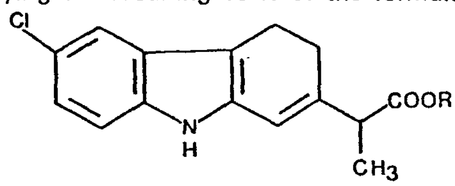

Ia

wherein R is as above,

c) if desired, converting the product of step a) or b) of the formula

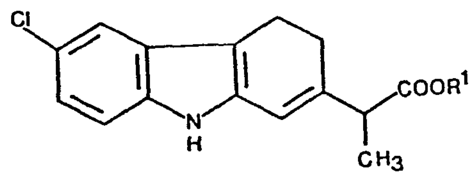

Ib

wherein $R^1$ is as above to 6 - chloro - 3,4 - dihydro - 2 - (2 - hydroxy - 1 - methylethyl) - carbazole of the formula

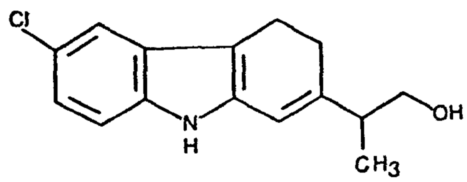

Ic

d) if desired aromatizing the product of step a) b) or c) of the formula

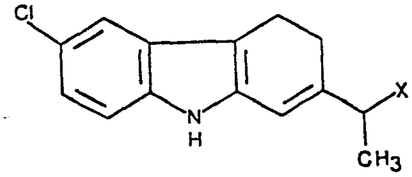

Id

wherein X is as above,

to a compound of formula I wherein the dotted line represents a C—C bond, and isolating an obtained compound of formula I, wherein X is carboxy, in that form or in form of a salt with a base.

The invention further relates to the novel intermediate, 6 - chloro - 1,2,3,4 - tetrahydrocarbazole - 2 - one.

Exemplary of the compounds of formula Id are:

6 - chloro - 3,4 - dihydrocarbazole - 2 - $\alpha$ - methylacetic acid;

6 - chloro - 3,4 - dihydrocarbazole - 2 - $\alpha$ - methylacetic acid ethyl ester;

6 - chloro - 3,4 - dihydrocarbazole - 2 - α - methylacetic acid methyl ester.

The reaction of the 6 - chloro - 1,2,3,4 - tetrahydro carbazole - 2 - one with a compound of formula II, e.g. with triethyl - α - methyl - phosphonoacetate, can be carried out in an inert organic solvent, e.g. a hydrocarbon, such as benzene, tetrahydrofuran or dimethylsulfoxide at or above room temperature, e.g. at a temperature in the range of from about 25°C to 100°C, preferably in the range or from about 25°C to 50°C. The reaction is carried out in the presence of a strong base, e.g. sodium hydride. The resulting 3,4 - dihydrocarbazole - α - methyl - 2 - acetic acid ester of formula Ia, can be recovered by conventional methods or reacted in situ in the next step of the process.

The ester of formula Ia can be saponified with an acid or base in a conventional manner.

An acid or ester of formula Ib can be converted to the corresponding alcohol of formula Ic by utilizing lithium aluminium hydride or sodium dihydrobismethoxyethoxyaluminate, and hydrolyzing the resulting product.

The aromatization of the 3,4-dihydrocarbazole of formula Id to the corresponding carbazole can be carried out e.g. with p-chloranil, o-chloranil, 2,3 - dichloro - 5,6 - dicyanobenzoquinone, sulfur or lead oxide in the presence of an inert organic solvent, e.g. xylene, benzene, toluene, quinoline, dimethylsulfoxide or dimethylformamide, preferably at the reflux temperature of the reaction mixture. The formed carbazole can be recovered in accordance with known procedures, e.g. by recrystallization.

The intermediate, 6 - chloro - 1,2,3,4 - tetrahydrocarbazole - 2 - one, can be prepared by reacting p - chloro - phenylhydrazine with dihydroresorcinol to yield a compound of the formula

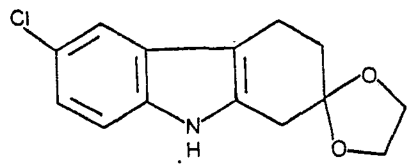

III

which is then deketalized utilizing known procedures. The reaction conditions are further described by R.F. Borch et al. in J. Org. Chem., *38*, 2729 (1973).

The compounds of formula I wherein the dotted line represents a C—C bond are known compounds useful as anti-inflammatory, analgesic and anti-rheumatic agents.

The compounds of formula Ib, when $R^1$ is hydrogen, forms salts with pharmaceutically acceptable bases, e.g. with alkali metal hydroxides or alkoxides, such as sodium or potassium hydroxide or ethylate; alkaline earth hydroxides, such as calcium or barium hydroxide; organic bases, such as piperidine, diethanolamine or N-methylglucamine.

The compounds of formula Id and the salts of the compound of formula Id, when X is carboxy, possess anti-inflammatory and anti-rheumatic properties. They also exhibit a significantly low incidence of ulcerogenic activity, which renders them highly desirable as anti-inflammatory and anti-rheumatic agents. Their pharmacologically useful activities are demonstrated in warm-blooded animals using standard procedures.

When 6 - chloro - 3,4 - dihydro - 2 - (2 - hydroxy - methyl - ethyl) - carbazole and 3,4 - dihydro - 6 - chloro - α - methyl - carbazole - 2 - acetic acid are utilized as test substances at a dosage of 0.03 mg. p.o. in rats, an anti-inflammatory activity is observed, i.e. the edemas are reduced, respectively, by 11.7 o/o and 37.6 o/o.

The compounds of formula Id have effects qualitatively similar to those of phenylbutazone and indomethacin, known for their therapeutic uses and properties.

The compounds of formula Id can be incorporated into standard pharmaceutical dosage forms, e.g. for oral or parenteral application, with usual pharmaceutical adjuvant material, e.g. organic or inorganic inert carrier materials such as water, gelatin, lactose, starch, magnesium stearate, talc, vegetable oils, gums or polyalkyleneglycols. The pharmaceutical preparations can be employed in a solid form, e.g. as tablets, troches, suppositories or capsules, or in liquid form, e.g. as solutions, suspensions or emulsions. Pharmaceutical adjuvant materials can be added, e.g. preservatives, stabilizers, wetting or emulsifying agents, salts to change the osmotic pressure or to act as buffers. The pharmaceutical preparations can also contain other therapeutically active substances.

Example 1

1) *Preparation of the starting material*

a) To a suspension of 17.90 g of p-chlorophenylhydrazine hydrochloride in 400 ml of toluene was added 13.4 g of dihydroresorcinol. The mixture was heated at reflux, with azeotropic removal of water under nitrogen for 10 minutes. The suspension was cooled slightly, treated with 50 ml of ethylene glycol and 2.28 g of p-toluenesulfonic acid monohydrate and heated for 4 hours. The mixture was cooled, treated with water and filtered. The organic layer was washed with water, saturated sodium bicarbonate solution and brine and dried. The material obtained upon solvent removal was crystallized from ether/petroleum ether to give 14.70 g of 6 - chloro - 2,2 - ethylenedioxy - 1,2,3,4 - tetrahydrocarbazole, mp 128—138°.

b) A suspension of 6 - chloro - 2,2 - ethylenedioxy - 1,2,3,4 - tetrahydrocarbazole in 200 ml of 1:1 acetic acid/water was heated at reflux under nitrogen for 1.5 hours. The mixture was filtered and the filtrate was cooled in an ice bath and again filtered. Crystallization of the solid from methylene chloride/petroleum ether gave 5.74 g (26 o/o yield) of 6 - chloro - 1,2,3,4 - tetrahydrocarbazole - 2 - one, mp. 194—196°.

2) *The Process*

a) 6.12 g of sodium hydride were suspended in 125 ml of benzene. A solution of 30.9 g of triethyl - α - methylphosphotoacetate in 125 ml of benzene was added over 1.0 hour to give a grey-green solution. 6.35 g of 6 - chloro - 1,2,3,4 - tetrahydrocarbazole - 2 - one was added in one portion and the mixture was heated at 40—45° for 1.5 hours. The reaction mixture was washed with 0.25 N hydrochloric acid, water and brine and dried. The material obtained upon solvent removal was chromatographed on silica gel with benzene/ethyl acetate and crystallized from ether/petroleum ether to give 6.35 g of racemic 6 - chloro - 3,4 - dihydrocarbazole - 2 - α - methylacetic acid ethyl ester, m.p. 101—102°.

b) A mixture of 1.52 g of racemic 6 - chloro - 3,4 - dihydrocarbazole - 2 - α - methylacetic acid ethyl ester and 1.84 g of chloranil in 50 ml of toluene was heated at reflux under nitrogen for 4.0 hours. The suspension was cooled, filtered, washed with 0.5N sodium hydroxide, water and brine, dried and stripped of solvent. The residual semi-solid was chromatographed on silica gel with benzene/ethyl acetate and crystallized from benzene/hexane to give 1.38 g of racemic 6 - chloro - α - methylcarbazole - 2 - acetic acid ethyl ester, mp 113—114°.

Example 2

To a solution of 3,03 g of 6 - chloro - 3,4 - dihydrocarbazole - 2 - α - methylacetic acid ethyl ester in 50 ml of ethanol was added 50 ml of 2N sodium hydroxide solution. The mixture was stirred 18 hours at 20° and acidified with 2N hydrochloric acid The resultant product was collected by filtration and crystallized from ether/petroleum ether to give 2.55 g of 6 - chloro - 3,4 - dihydrocarbazole - 2 - α - methylacetic acid as a white solid, mp 140—141° (dec.).

Example 3

To an ice cold solution of 303 mg of 6 - chloro - 3,4 - dihydrocarbazole - 2 - α - methylacetic acid ethyl ester in 10 ml of tetrahydrofuran was added over 15 minutes 2 ml of a 70% solution of sodium dihydro-bis(2-methoxyethoxy)aluminate in benzene. The mixture was stirred at 3° for 90 minutes and treated with 5 ml of methanol, 15 ml of water and 50 ml of ether. The organic phase was washed with 0,5N hydrochloric acid, water and brine, dried and stripped of solvent to give an oil. Two crystallizations of this material from ether/petroleum ether gave 153 mg of 6 - chloro - 3,4 - dihydro - 2 - (2 - hydroxy - 1 - methylethyl) - carbazole, mp 126—127°.

Preparation of Pharmaceutical Compositions

1) Suppositories of the following composition are manufactured in conventional manner:

| | |
|---|---|
| 6-chloro-3,4-dihydrocarbazole-α-methyl-2-acetic acid | 0,025 g |
| Hydrogenated coconut oil | 1,230 g |
| Carnauba wax | 0,045 g |

2) Tablets of the following composition are manufactured in conventional manner:

| | |
|---|---|
| 6-chloro-3,4-dihydrocarbazole-α-methyl-2-acetic acid | 25,00 mg |
| Lactose | 64,50 mg |
| Corn starch | 10,00 mg |
| Magnesium Stearate | 0,50 mg |

3) Capsules of the following composition are manufactured in conventional manner:

| | |
|---|---|
| 6-chloro-3,4-dihydrocarbazole-α-methyl-2-acetic acid | 50 mg |
| Lactose | 124 mg |
| Corn starch | 30 mg |
| Talc | 5 mg |

4) Parenteral Formulation
Each 1 ml ampule contains:

| | |
|---|---|
| 6-chloro-3,4-dihydrocarbazole-α-methyl-2- | 10,2 mg |
| acetic acid | (2% excess) |
| 4-Hydroxybenzoic acid methyl ester | 1,8 mg |
| 4-Hydroxybenzoic acid propyl ester | 0,2 mg |
| Sodium hydroxide q.s. ph | 9,0 |
| Water q.s. ad | 1 ml |

**Claims for Contracting States: CH, LU, NL, SE**

1. Process for the preparation of carbazole derivates of formula

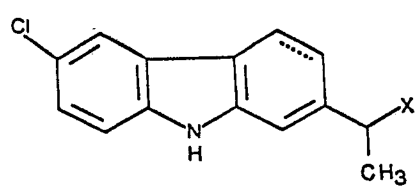

I

wherein X is hydroxymethyl or a grouping $COOR^1$, $R^1$ is hydrogen or lower alkyl, and the dotted line denotes an optional C—C bond, and, where X is carboxy, salts thereof with bases, comprising

a) reacting 6 - chloro - 1,2,3,4 - tetrahydro - carbazole - 2 - one with a trialkyl - α - methylphosphonoacetate of the formula

$$(RO)_2\overset{O}{\overset{\uparrow}{P}}—\overset{CH_3}{\overset{|}{C}}HCOOR \qquad II$$

wherein R is lower alkyl, in the presence of a strong base,

b) if desired, saponifying the resulting ester of the formula

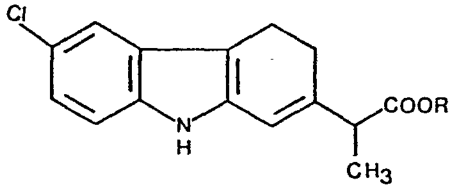

Ia

wherein R is as above,

c) if desired, converting the product of step a) or b) of the formula

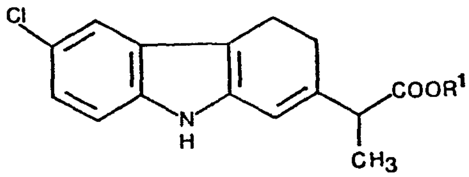

Ib

wherein $R^1$ is as above,

to 6 - chloro - 3,4 - dihydro - 2 - (2 - hydroxy - 1 - methylethyl) - carbazole of the formula

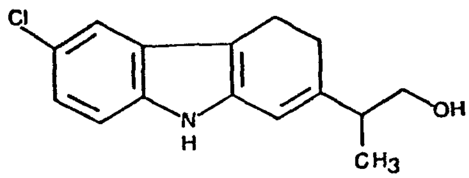

Ic

d) if desired aromatizing the product of step a), b) or c) of the formula

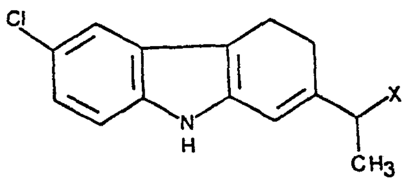

Id

wherein X is as above,

to a compound of formula I wherein the dotted line represents a C—C bond, and isolating an obtained compound of formula I, wherein X is carboxy, in that form or in form of a salt with a base.

2. 6 - Chloro - 1,2,3,4 - tetrahydro carbazole - 2 - one.

**Patentansprüche für die Vertragsstaaten: CH, LU, NL, SE**

1. Verfahren zur Herstellung von Carbazol-Derivaten der Formel

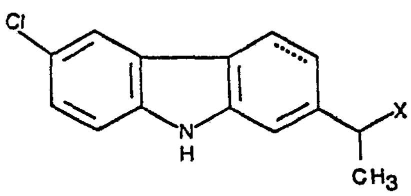

I

worin X Hydroxymethyl oder eine Gruppierung $COOR^1$, $R^1$ Wasserstoff oder Niederalkyl ist und die gestrichelte Linie eine mögliche C—C-Bindung bezeichnet und wenn X Carboxy ist, deren Salzen mit Basen, unter

a) Umsetzen von 6 - Chlor - 1,2,3,4 - tetrahydro - carbazol - 2 - on mit einem Trialkyl - $\alpha$ - methylphosphonoacetat der Formel

$$\begin{array}{c} O \quad CH_3 \\ \uparrow \quad | \\ (RO)_2P\text{—}CHCOOR \end{array} \qquad II$$

worin R Niederalkyl ist, in Gegenwart einer starken Base,

b) wenn gewünscht, Verseifen des anfallenden Esters der Formel

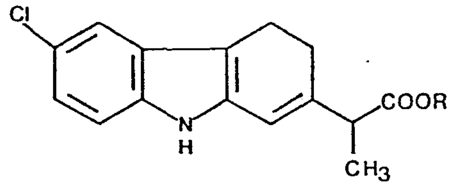

Ia

worin R wie oben ist,

c) wenn gewünscht, Umwandeln des Produkts der Stufe a) oder b) der Formel

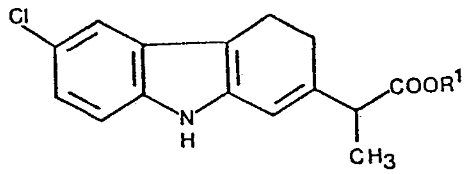

Ib

worin $R^1$ wie oben ist, in 6 - Chlor - 3,4 - dihydro - 2 - (2 - hydroxy - 1 - methyläthyl) - carbazol der Formel

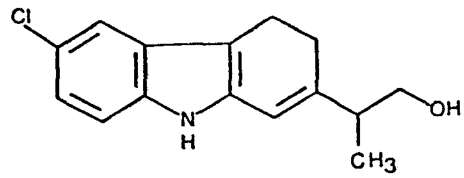

Ic

d) wenn gewünscht, Aromatisieren des Produkts der Stufe a), b) oder c) der Formel

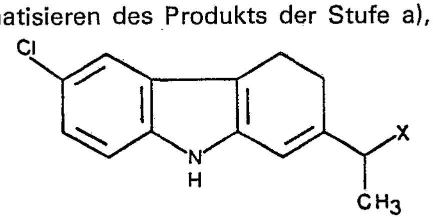

Id

worin X wie oben ist, in eine Verbindung der Formel I, worin die gestrichelte Linie eine C—C-Bindung bedeutet, und Isolieren einer erhaltenen Verbindung der Formel I, worin X Carboxy ist, in der Form oder in Form eines Salzes mit einer Base.

2. 6-Chlor-1,2,3,4-tetrahydro-carbazol-2-on.

**Revendications pour les états contractants: CH, LU, NL, SE**

1. Procédé de préparation de dérivés de carbazole de formule

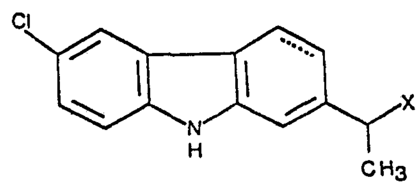

I

dans laquelle X est un groupe hydroxyméthyle ou un groupement $COOR^1$, $R^1$ est de l'hydrogène ou un groupe alcoyle inférieur et la ligne en pointillé indique une liaison C—C éventuelle, et, quand X est un groupe carboxy, leurs sels avec des bases, caractérisé en ce que

a) on fait réagir la 6 - chloro - 1,2,3,4 - tétrahydrocarbazole - 2 - one avec un trialcoyl - $\alpha$ - méthylphosphonoacétate de la formule

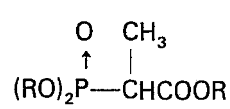

II

dans laquelle R est un groupe alcoyle inférieur, en présence d'une base forte,

b) si on le désire, on saponifie l'ester résultant de la formule

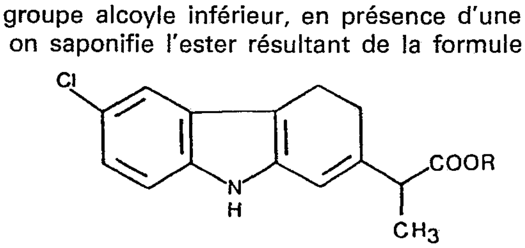

Ia

dans laquelle R est tel que ci-dessus,

c) si on le désire, on transforme le produit de l'étape a) ou b) de la formule

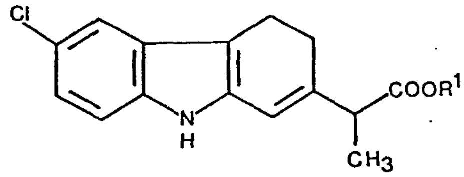

Ib

dans laquelle $R^1$ est tel que ci-dessus, en 6 - chloro - 3,4 - dihydro - 2 - (2 - hydroxy - 1 - méthyléthyl)carbazole de la formule

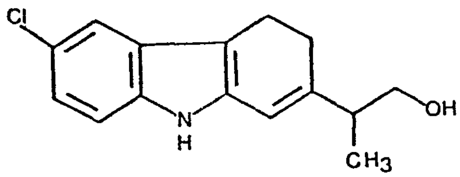

Ic

d) si on le désire, on aromatise le produit de l'étape a), b) ou c) de la formule

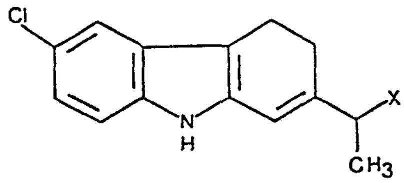

Id

dans laquelle X est tel que ci-dessus, de manière à obtenir un composé de formule I dans lequel la ligne en pointillé représente une liaison C—C, et on isole un composé obtenu de formule I, dans lequel X est un groupe carboxy, sous cette forme ou sous la forme d'un sel avec une base.

2. 6-chloro-1,2,3,4-tétrahydrocarbazole-2-one.

**Claim for Contracting State: AT**

1. Process for the preparation of carbazole derivatives of formula

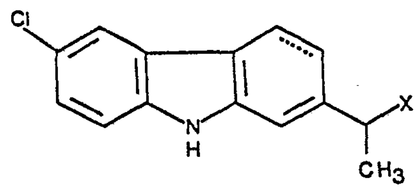

I

wherein X is hydroxymethyl or a grouping $COOR^1$, $R^1$ is hydrogen or lower alkyl, and the dotted line denotes an optional C—C bond, and, where X is carboxy, salts thereof with bases, comprising

a) reacting 6 - chloro - 1,2,3,4 - tetrahydro - carbazole - 2 - one with a trialkyl - $\alpha$ - methylphosphonoacetate of the formula

$$(RO)_2\overset{\overset{O}{\uparrow}}{P}—\overset{\overset{CH_3}{|}}{C}HCOOR \qquad II$$

wherein R is lower alkyl,
in the presence of a strong base,

b) if desired, saponifying the resulting ester of the formula

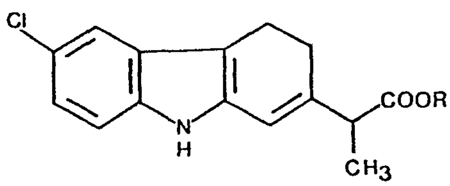

Ia

wherein R is as above,

c) if desired, converting the product of step a) or b) of the formula

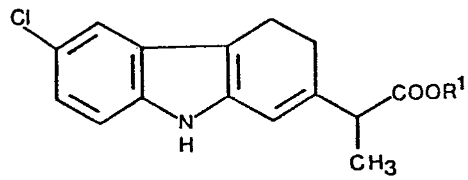

Ib

wherein $R^1$ is as above,

to 6 - chloro - 3,4 - dihydro - 2 - (2 - hydroxy - 1 - methylethyl) - carbazole of the formula

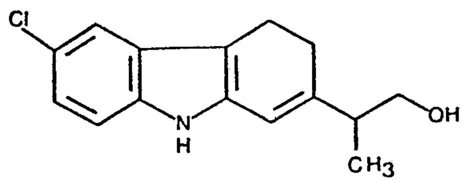

Ic

d) if desired aromatizing the product of step a), b) or c) of the formula

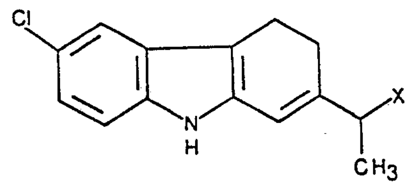

Id

wherein X is as above,

to a compound of formula I wherein the dotted line represents a C—C bond, and isolating an obtained compound of formula I, wherein X is carboxy, in that form or in form of a salt with a base.

**Patentanspruch für den Vertragsstaat: AT**

1. Verfahren zur Herstellung von Carbazol-Derivaten der Formel

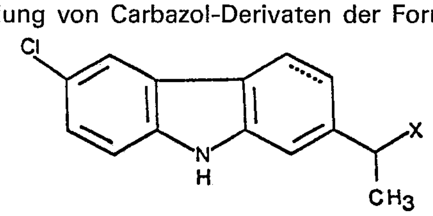

I

worin X Hydroxymethyl oder eine Gruppierung $COOR^1$, $R^1$ Wasserstoff oder Niederalkyl ist und die gestrichelte Linie eine mögliche C—C-Bindung bezeichnet und wenn X Carboxy ist, deren Salzen mit Basen, unter

a) Umsetzen von 6 - Chlor - 1,2,3,4 - tetrahydro - carbazol - 2 - on mit einem Trialkyl - $\alpha$ - methylphosphonoacetat der Formel

$$\begin{array}{cc} O & CH_3 \\ \uparrow & | \\ (RO)_2P & \!\!\!\!—CHCOOR \end{array} \qquad II$$

worin R Niederalkyl ist, in Gegenwart einer starken Base,

b) wenn gewünscht, Verseifen des anfallenden Esters der Formel

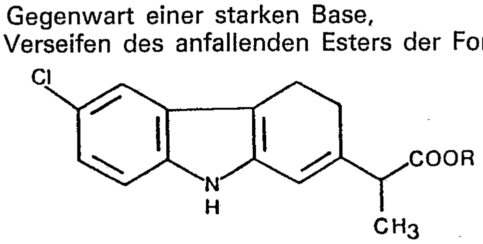

Ia

worin R wie oben ist,

c) wenn gewünscht, Umwandeln des Produkts der Stufe a) oder b) der Formel

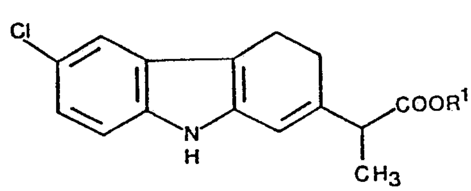

Ib

worin $R^1$ wie oben ist, in 6 - Chlor - 3,4 - dihydro - 2 - (2 - hydroxy - 1 - methyläthyl) - carbazol der Formel

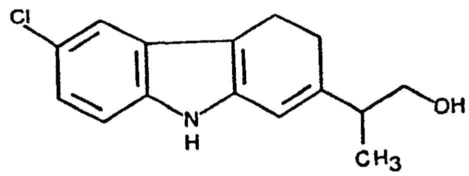

Ic

d) wenn gewünscht, Aromatisieren des Produkts der Stufe a), b) oder c) der Formel

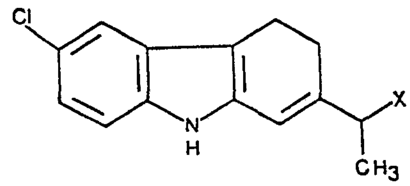

Id

worin X wie oben ist, in eine Verbindung der Formel I, worin die gestrichelte Linie eine C—C-Bindung bedeutet, und Isolieren einer erhaltenen Verbindung der Formel I, worin X Carboxy ist, in der Form oder in Form eines Salzes mit einer Base.

**Revendication pour l'état contractant: AT**

1. Procédé de préparation de dérivés de carbazole de formule

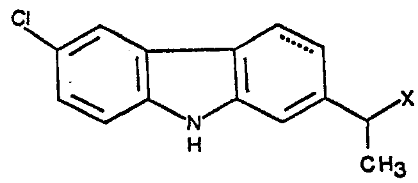

I

dans laquelle X est un groupe hydroxyméthyle ou un groupement $COOR^1$, $R^1$ est de l'hydrogène ou un groupe alcoyle inférieur et la ligne en pointillé indique une liaison C—C éventuelle, et, quand X est un groupe carboxy, leurs sels avec des bases, caractérisé en ce que

a) on fait réagir la 6 - chloro - 1,2,3,4 - tétrahydrocarbazole - 2 - one avec un trialcoyl - $\alpha$ - méthylphosphonoacétate de la formule

O $CH_3$
↑ |
$(RO)_2P$—CHCOOR

II

dans laquelle R est un groupe alcoyle inférieur, en présence d'une base forte,

b) si on le désire, on saponifie l'ester résultant de la formule

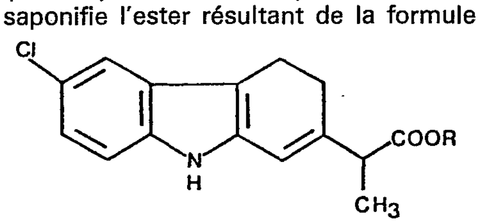

Ia

dans laquelle R est tel que ci-dessus,

c) si on le désire, on transforme le produit de l'étape a) ou b) de la formule

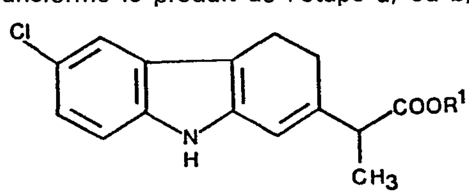

Ib

dans laquelle $R^1$ est tel que ci-dessus, en 6 - chloro - 3,4 - dihydro - 2 - (2 - hydroxy - 1 - méthyléthyl)carbazole de la formule

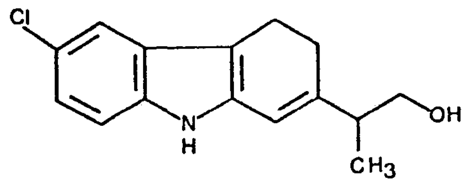

Ic

d) si on le désire, on aromatise le produit de l'étape a), b) ou c) de la formule

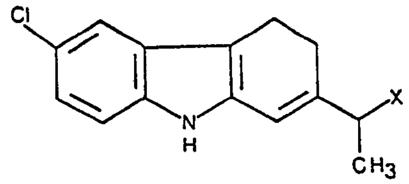

Id

dans laquelle X est tel que ci-dessus, de manière à obtenir un composé de formule I dans lequel la ligne en pointillé représente une liaison C—C, et on isole un composé obtenu de formule I, dans lequel X est un groupe carboxy, sous cette forme ou sous la forme d'un sel avec une base.